# FASCICULE DE BREVET EUROPEEN

(11) **EP 0 502 939 B1**
(45) Date de publication et mention de la délivrance du brevet: **02.02.1994**
(21) Numéro de dépôt: 91900268.3
(22) Date de dépôt: 26.11.1990
(51) Int. Cl.: A61B 19/00

(54) **DISPOSITIF POUR ISOLER SOUS UN FLUX LIQUIDE UN CHAMP OPERATOIRE CHIRURGICAL**
GERÄT ZUR ISOLIERUNG EINER CHIRURGISCHEN OPERATIONSSTELLE IN EINER FLÜSSIGKEITSSTRÖMUNG
DEVICE FOR INSULATING A SURGICAL OPERATION AREA UNDER A FLOW OF LIQUID

(30) Priorité: 28.11.1989 FR 8915946
(43) Date de publication de la demande: 16.09.1992
(73) Titulaire: MIROUZE, Alain, F-13127 Vitrolles (FR)
(72) Inventeur: MIROUZE, Alain, F-13127 Vitrolles (FR)
(74) Mandataire: Roman, Michel
(86) Numéro de dépôt international: FR9000848
(87) Numéro de publication internationale: WO9107921

(56) Documents cités:
- FR-A- 2 570 596
- GB-A- 617 305
- GB-A- 1 118 657
- GB-A- 1 475 078
- US-A- 4 767 405

## Description

La présente invention a pour objet un dispositif pour isoler sous un flux liquide un champ opératoire chirurgical.

Elle concerne le remplacement ou le complément des salles d'opérations chirurgicales et peut être utilisée dans certains traitements médicaux tel que le soin des grands brûlés.

Dans les salles d'opérations courantes, la nécessité d'assurer des conditions d'aseptie absolue de l'environnement entraine la mise en oeuvre de très gros moyens, complexes et coûteux, demandant un temps de préparation important, ce qui peut s'avérer extrémement gênant pour des interventions en urgence, par exemple. De plus, l'aseptie réelle est très rarement assurée de façon satisfaisante.

Le brevet FR-A-2570 596 du même inventeur, décrit un bac étanche permettant de remédier au moins en partie à cet état de chose en immergeant la partie à opérer dans un liquide aseptique en circulation permanente et en effectuant l'intervention au-dessous du niveau du liquide. Ce dispositif est surtout appliquable pour des interventions d'importance limitée et ne peut s'utiliser directement que pour des opérations sur les extrémités des membres. Toutefois, le brevet précité revendique également un bac pouvant être traversé par le membre à opérer grâce à des ouvertures latérales opposées, ce qui permet d'intervenir sur la partie supérieure dudit membre, et éventuellement sur le tronc, mais ce dernier cas nécessite un matériel très volumineux. Ce système présente par ailleurs des problèmes visuels dus aux reflets provoqués par l'éclairage scialitique sur la surface du liquide, ainsi que par l'obscurcissement et à la pollution dus aux tourbillons créés par la circulation du liquide.

Le dispositif suivant la présente invention supprime ces inconvé nients. En effet, il permet des interventions de toutes importances, aussi bien sur le tronc que sur les membres, avec des moyens limités, des temps de préparation réduits et de bonnes conditions de visibilité.

Il est constitué par la combinaison des éléments indiqués dans la partie caractérisante de la revendication 1.

Sur les dessins annexés, donnés à titre d'exemple non limitatif d'une des formes de réalisation de l'objet de l'invention :

La figure 1 représente en perspective l'ensemble du dispositif.

La figure 2 est une coupe transversale à une autre échelle suivant les flèches A-A de la figure 1.

La figure 3 est une vue de dessus.

Les figures 4 et 5 sont des coupes verticales, longitudinales et transversales, respectivement suivant les flèches B-B et C-C de la figure 3, montrant l'emplacement d'une source lumineuse. Les figures 6 et 7 sont des coupes verticales, longitudinales et transversales, respectivement suivant les flèches B-B et C-C de la figure 3, montrant la disposition des instruments chirurgicaux.

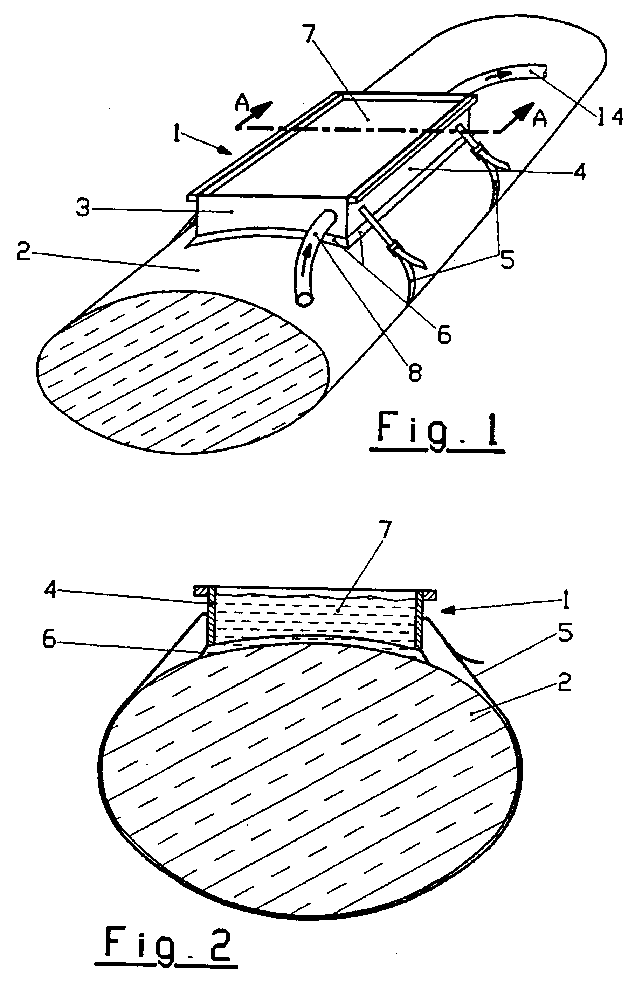

Le dispositif, figure 1 à 7, est constitué d'un bac 1 dépourvu de fond reposant sur la zone à opérer 2 en la délimitant, la pression exercée par ses parois latérales 3, 4 sur la peau assurant l'étanchéité de l'ensemble, complétée éventuellement par des attaches ou sangles 5. Le cas échéant, des lèvres souples 6 peuvent compléter cette étanchéité.

Diverses tailles et formes de bacs sans fond peuvent être créées pour s'adapter aux différentes parties du corps.

Un flux laminaire permanent de liquide aseptique 7 est entretenu à l'intérieur du bac au-dessus de la partie à opérer.

En effet, ce flux doit être aussi régulier que possible afin d'éviter les remous susceptibles de faire retourner des pollutions sur la zone d'intervention. Pour obtenir ce résultat le flux de liquide arrive par un tube 8 dans une chambre de tranquilisation 9, passe à travers une grille ou filtre d'entrée 10 ou structure fine en alvéoles (par exemple en nid d'abeille) disposée sur toute la largeur du bac de façon à ce que les vitesses d'écoulement soient uniformes sur la face de sortie. Si nécessaire la grille ou filtre peut être complétée par une ou plusieurs autres grilles ou filtres similaires 11. A l'autre extrémité du bac 1 une grille ou filtre de sortie 12 de même nature est traversée par le liquide qui arrive ensuite dans une seconde chambre de tranquilisation 13, puis s'écoule par le tube 14.

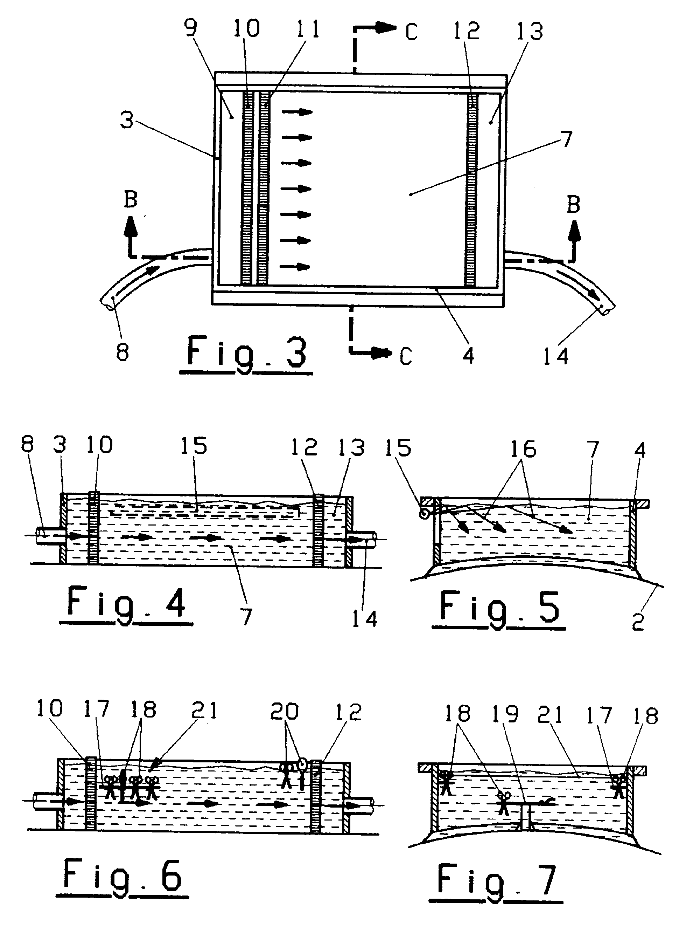

En dépit de l'excellence des technologies actuelles, les reflets dus à l'éclairage par le dessus de la surface du liquide constituent une gêne non négligeable. C'est pourquoi l'éclairage du champ opératoire peut être avantageusement cbtenu par une ou plusieurs sources lumineuses 15 (fig 4 et 5) disposées latéralement à l'extérieur du bac 1, de préférence parallèlement à la direction du flux du liquide, en dessous du niveau du liquide aseptique 7, assez près de la surface et en regard d'une partie transparente des parois 3, 4. En effet, il est connu que, du fait de la différence d'indice de réfraction, la surface d'un liquide joue le rôle d'un miroir sous certains angles d'incidence de la lumière (flèches 16, figure 5).

Une autre possibilité consiste à placer directement la ou les sources lumineuses à l'intérieur du bac 1 avec une localisation semblable.

Les parois 3, 4 du bac 1 peuvent comporter des supports internes 17 permettant de maintenir les instruments chirurgicaux 18 dans le liquide aseptique 7. D'autres supports 19, destinés plus particulièrement aux pinces de fixation et aux dispositifs de contention peuvent être fixés aux emplacements les plus opportuns, à l'intérieur de la zone liquide.

Certains instruments chirurgicaux 20 sont munis de moyens de flottaison disposés de manière à ce que la partie de l'instrument entrant en contact l'opéré baigne en permanence dans le liquide aseptique 7. Un fil 21 (ou une tige) tendu près de la surface du liquide, parallèlement aux grilles d'entrée et de sortie 10, 12 délimite une zone en amont pour les instruments propres, les autres dérivant vers la grille de sortie 12.

L'utilisation de liquide spécifique permet de diminuer dans des proportions importantes la nécessité de réhydratation des malades effectuée habituellement par perfusion post-opératoire.

Le liquide opératoire peut également être additionné de substances diverses permettant une diminution des effets du choc opératoire et un apport ionique correspondant aux pertes prévisibles lors de l'intervention.

Selon un procédé original, il est possible de traiter un grand brûlé en le réhydratant et le nourrissant grâce au bain immergeant l'ensemble de la partie brûlée. Le liquide 7 circulant dans ce cas est constamment enrichi de substances nutritives et de traitement et détoxiqué lors de son passage dans l'appareil de traitement et de mise en circulation du liquide.

Le positionnement des divers éléments constitutifs donnent à ce dispositif un maximum d'effets utiles qui n'avaient pas été obtenus à ce jour par des dispositifs similaires.

## Revendications

1. Dispositif pour isoler sous un flux liquide un champ opératoire chirurgical, destiné au remplacement ou au complément des salles d'opérations chirurgicales, mais pouvant être util'isé dans certains traitements médicaux tel que le soin des grands brûlés, consistant à immerger la partie à opérer ou à traiter dans un liquide aseptique en circulation permanente et en effectuant l'intervention au-dessous du niveau du liquide caractérisé par la combinaison d'un bac (1) sans fond posé à même la peau du patient (2), de dimensions telles que les parois latérales (3, 4) entourent la partie à opérer, et dans lequel est maintenu un flux laminaire permanent, mais réglable et modulable, de liquide aseptique (7) provenant d'un appareil connu de traitement et de mise en circulation dudit liquide lequel,arrivant par un tube (8) dans une chambre de tranquilisation (9), passe ensuite à travers une ou plusieurs grilles ou filtres d'entrée (10, 11) ou structures fines en alvéoles (par exemple en nid d'abeille) disposées sur toute la largeur du bac de façon à ce que les vitesses d'écoulement soient uniformes sur la face de sortie, à l'autre extrémité du bac (1) une grille ou filtre de sortie (12) de même nature est traversée par le liquide qui arrive ensuite dans une seconde chambre de tranquilisation (13) puis s'écoule hors du bac (1) par un tube (14) l'éclairage de la zone d'intervention étant assuré par une ou plusieurs sources lumineuses (15) disposées en dessous du niveau du liquide aseptique (7), assez près de la surface, de façon que les rayons réfléchis par la surface du liquide soient dirigés sur la zone de travail (flèches 16)

2. Dispositif suivant la revendication 1, se caractérisant par le fait que l'étanchéité est assurée par des moyens tels que sangles (5) appliquant le bac contre la peau et des lèvres souples (6) prévues sur le pourtour inférieur dudit bac.

3. Dispositif suivant l'une quelconque des revendications précédentes, se caractérisant par le fait que l'éclairage de la zone d'intervention est assuré par une ou plusieurs sources lumineuses (15) disposées latéralement à l'extérieur du bac (1), en regard d'une partie transparente des parois (3, 4) de celui-ci.

4. Dispositif suivant l'une quelconque des revendications 1 et 2, se caractérisant par le fait que la ou les sources lumineuses (15) sont placées à l'intérieur du bac (1), sous le niveau du liquide aseptique (7).

5. Dispositif suivant l'une quelconque des revendications précédentes, se caractérisant par le fait que les parois (3, 4) du bac (1) comportent des supports internes (17) permettant de maintenir les instruments chirurgicaux (18) dans le liquide aseptique (7), d'autres supports (19), destinés plus particulièrement aux pinces de fixation et aux dispositifs de contention étant fixés à l'intérieur de la zone liquide.

6. Dispositif suivant l'une quelconque des revendications précédentes, se caractérisant par le fait que certains instruments chirurgicaux (20) sont munis de moyens de flottaison disposés de manière à ce que la partie de l'instrument entrant en contact avec l'opéré baigne en permanence dans le liquide aseptique (7), un fil (21) (ou une tige) tendu près de la surface du liquide, parallèlement aux grilles ou filtres d'entrée et de sortie (10, 12) délimitant une zone en amont pour les instruments propres, les autres dérivant vers la grille ou filtre de sortie (12).

## Claims

1. Device which, under a liquid flux, isolates a surgical operating field and is designed to replace or to complement operating theaters, but which can be used for certain medical treatments such as serious burns, and which consists in immersing the part to be operated or to be treated in a continuously circulating aseptic liquid and carrying out the surgical intervention below the level of the liquid, characterized by the combination of a bottomless tank (1) laid against the patient's skin (2) and dimensioned so that the side walls (3, 4) enclose the region to be operated and in which there is a continuous adjustable and modulable laminar flux of aseptic liquid (7) drawn from a known apparatus for treatment and circulation of the said liquid which, flowing into a settling chamber (9) through a tube (8) then passes through one or several inlet grills or filters (10, 11) or fine-cell structures (for instance honeycombed) arranged across the width of the tank so that the rates of flow are uniform on the outlet face, and at the other end of the tank (1) the liquid flows through a grill or outlet filter (12) of the same type to reach a second settlement chamber (13), and then flow outside the tank (1) through a tube (14), lighting of the operating area being provided by one or several light sources (15) arranged above the level of the aseptic liquid (7) fairly close to the surface so that the rays reflected by the surface of the liquid are directed onto the work area (arrows 16).

2. Device as per claim 1 characterized by the leaktightness being provided by means such as belts (5) applying the tank against the skin and flexible lips (6) around the lower periphery of the said tank.

3. Device as per any one of the aforesaid claims, characterized by the lighting of the operating area being provided by one or several lights (15) arranged laterally on the outside of the tank (1) opposite a transparent part of the walls (3, 4) of the latter.

4. Device as per any one of claim 1 and 2 characterized by the light source(s) (15) being positioned inside the tank (1) under the level of the aseptic liquid (7).

5. Device as per any one of the aforesaid claims characterized by the walls (3, 4) of the tank (1) including internal supports (17) allowing surgical instruments (18) to be kept in the aseptic liquid (7), the other supports (19) being more particularly used for securing clips and containers inside the liquid area.

6. Device as per any one of the aforesaid claims characterized by certain surgical instruments (20) being fitted with floats arranged so that the parts of the instrument entering into contact with the patient are permanently bathed in the aseptic liquid (7), a wire (21) (or rod) held tight close to the surface of the liquid, parallel to the grills or inlet and outlet filters (10, 12) forming the boundary of an upstream area for clean instruments, the others drifting towards the grill or outlet filter (12).

## Patentansprüche

1. Vorrichtung zur Isolierung eines chirurgischen Operationsfeldes unter einer Flüssigkeitsströmung, dazu bestimmt, chirurgische Operationssäle zu ersetzen oder zu ergänzen, aber auch zur Verwendung für gewisse medizinische Behandlungen wie z.B. die Behandlung von schweren Verbrennungen, die darin besteht die zu operierende bzw. zu behandelnde Körperpartie in eine aseptische, in ständigem Kreislauf befindliche Flüssigkeit zu tauchen und den Eingriff unter dem Flüssigkeitspegel vorzunehmen, gekennzeichnet durch die Kombination eines Beckens (1) ohne Boden, unmittelbar auf der Haut des Patienten angebracht und von solcher Größe, daß die Seitenwände (3, 4) die zu operierende Körperpartie einschließen, und innerhalb denen eine ständige laminare, jedoch regelbare und modulierbare Strömung einer aseptischen Flüssigkeit (7) herrscht, aus einem bekannten Behandlungsgerät kommend, welches die besagte Flüssigkeit in Strömung versetzt, die durch einen Schlauch (8) in eine Beruhigungskammer (9) einfließt, dann durch ein oder mehrere Einlaufgitter bzw. -filter (10, 11) oder feine (z.B. wabenförmige) über die gesamte Breite des Beckens angeordnete Zellstrukturen strömt, sodaß die Strömungsgeschwindigkeiten auf der Ausgangsseite ausgeglichen sind, am anderen Ende des Beckens (1) ein Auslaufgitter bzw. -filter (12) der gleichen Art angeordnet ist, durch welches die Flüssigkeit in eine zweite Beruhigungskammer (13) kommt und dann durch den Schlauch (14) aus dem Becken (1) abfließt, die Beleuchtung des Eingriffsbereichs wird dabei durch eine oder mehrere Lichtquellen (15) gewährleistet, welche unterhalb des Pegels der aseptischen Flüssigkeit (7) angeordnet sind, jedoch nahe genug an der Oberfläche, damit die von der Oberfläche reflektierten Strahlen auf den Arbeitsbereich gerichtet sind (Pfeile 16).

2. Vorrichtung nach Anspruch 1, dadurch gekennzeichnet, daß die Dichtheit durch Mittel wie Gurte (5), welche das Bekken auf die Haut drücken, und biegsamen auf dem unteren Umfang des besagten Beckens vorgesehenen Lippen (6) gewährleistet wird.

3. Vorrichtung nach irgendeinem der vorstehenden Ansprüche, dadurch gekennzeichnet, daß die Beleuchtung des Eingriffsbereichs durch eine oder mehrere seitlich außerhalb des Beckens (1) auf der Höhe eines transparenten Teils seiner Seitenwände (3, 4) angeordneten Lichtquellen (15) gewährleistet wird.

4. Vorrichtung nach irgendeinem der Ansprüche 1 und 2, dadurch gekennzeichnet, daß die Lichtquelle(n) (15) im Innern des Beckens (1) unterhalb des Pegels der aseptischen Flüssigkeit (7) angeordnet ist/sind.

5. Vorrichtung nach irgendeinem der vorstehenden Ansprüche, dadurch gekennzeichnet, daß die Wandungen (3, 4) des Bekkens (1) interne Halterungen (17) aufweisen, welche erlauben, chirurgische Instrumente (18) in der aseptischen Flüssigkeit zu halten, und andere Träger (19), bestimmt insbesondere für Haltezangen und Fixiervorrichtungen innerhalb des flüssigen Bereichs angebracht sind.

6. Vorrichtung nach irgendeinem der vorstehenden Ansprüche, dadurch gekennzeichnet, daß gewisse chirurgische Instrumente (20) mit Schwimmervorrichtungen versehen sind, welche so angeordnet sind, daß der Teil des Instrumentes, der mit dem Patienten in Kontakt kommt, ständig in die aseptische Flüssigkeit (7) getaucht ist, ein Faden (oder ein Draht) die obere Zone für die sauberen Instrumente abgrenzt, während die anderen zum Auslaufgitter bzw. -filter (12) abtreiben.
